# EUROPÄISCHE PATENTANMELDUNG

(11) **EP 2 431 352 A2**
(43) Veröffentlichungstag der Anmeldung: **21.03.2012**
(21) Anmeldenummer: 11175402.4
(22) Anmeldetag: 26.07.2011
(51) Int. Cl.: C07C 67/03, C11C 3/00, C07C 69/52

(54) **Katalysatorsysteme für die Biodieselherstellung**

(30) Priorität: 17.09.2010 DE 102010040939
(71) Anmelder: Evonik Degussa GmbH, 45128 Essen (DE)
(72) Erfinder: Ruwwe, Johannes, 53859 Niederkassel (DE); Lichtenheldt, Martin, 51145 Köln (DE); Orlia, Wolfgang-Wilhelm, 47918 Tönisvorst (DE)

(57) **Zusammenfassung**

Die vorliegende Erfindung betrifft die Verwendung eines Katalysatorsystems umfassend einen Umesterungskatalysator, ausgewählt aus der Gruppe der Alkalimetall- oder Erdalkalimetall-alkanolate oder der Alkalimetallhydroxide, und mindestens einem vom Umesterungskatalysator verschiedenen Aktivator, ausgewählt aus der Gruppe umfassend salzartige Verbindungen, Titanate oder nicht salzartige Verbindungen mit einer Dichte von mindestens 0.9 g/mL, zur Katalyse von Umesterungsreaktionen.

## Beschreibung

Die vorliegende Erfindung betrifft die Verwendung eines Katalysatorsystems umfassend einen Umesterungskatalysator, ausgewählt aus der Gruppe der Alkalimetall- oder Erdalkalimetall-alkanolate oder der Alkalimetallhydroxide, und mindestens einem vom Umesterungskatalysator verschiedenen Aktivator, ausgewählt aus der Gruppe umfassend salzartige Verbindungen, Titanate oder nicht salzartige Verbindungen mit einer Dichte von mindestens 0,9 g/mL, zur Katalyse von Umesterungsreaktionen.

Fettsäurealkylester einwertiger Alkohole finden seit einiger Zeit eine wichtige Anwendung in der Verwendung als Biodiesel, einem auf nachwachsenden Rohstoffen basierendem Ersatz für fossilen Diesel.

Die Herstellung von Biodiesel erfolgt in der Regel mittels basenkatalysierter Umesterung (The Biodiesel Handbook, G. Knothe, J. van Gerpen, J. Krahl, Ed. AOCS Press (2005); Biodiesel - The comprehensive handbook, M. Mittelbach, C. Remschmidt (2004); Bioresource Technology 2004, 92, 297; Applied Energy 2010, 87, 1083; Chimica Oggi/Chemistry today 2008, 26).

Die häufigsten verwendeten Katalysatoren sind Natrium-methanolat (NaOMe), Natrium-hydroxid (NaOH), Kalium-methanolat (KOMe) und Kalium-hydroxid (KOH). Diese Katalysatoren werden üblicherweise als homogene Katalysatoren, gelöst in dem verwendeten einwertigen Alkohol, wie z. B. Methanol, eingesetzt.

Alternativ dazu ist beschrieben, dass alkalische Katalysatoren in Verbindung mit Phasentransferkatalysatoren eingesetzt werden können (WO 2007/111604). Die Phasentransferkatalysatoren sorgen dafür, dass, verglichen mit dem Einsatz des alkalischen Katalysators ohne Phasentransferkatalysator, die Reaktion beschleunigt und ein vollständigerer Umsatz erzielt wird. Nachteilig an dem beschriebenen Verfahren ist, dass die Phasentransferkatalysatoren teuer und häufig korrosiv sind, weil sie Chlorid, Bromid oder andere Ionen enthalten, gegen die die Stahlreaktoren nicht beständig sind, in denen üblicherweise Biodiesel hergestellt wird. Weiterhin besteht die Gefahr, dass sich kleine Mengen des Phasentransferkatalysators nicht durch Aufarbeitung des Biodiesels aus diesem entfernen lassen.

Eine andere Möglichkeit die Reaktionsmischung zu modifizieren ist in den Offenlegungen DE 332506, DE 3415529, DE 102006044467 und DE 102007056703 genannt, wobei offenbart wird, dass ein Teil der im Umesterungsprozess erhaltenen Glycerinmenge zurückgeführt und der Reaktionsmischung aus Triglycerid, einwertigem Alkohol und alkalischen Katalysator beigemischt wird. Dieses Verfahren erlaubt die Einsparung des Katalysators, nachteilig daran ist jedoch, dass durch Zugabe von Glycerin das Gleichgewicht der Umesterungsreaktion auf die Seite der Edukte verschoben wird und unter Umständen kein ausreichender Umsatz erzielt wird.

Weiterhin ist die Möglichkeit beschrieben, den Prozess zur Herstellung von Biodiesel dadurch zu verbessern, dass der Reaktionsmischung nach der Umesterung Additive zugegeben werden, mit denen die Phasentrennung zur Abtrennung des freigesetzten Glycerins beschleunigt wird.

Eur. J. Lipid Sci. Technol. 2008, 110, 347 beschreibt die Zugabe von Wasser zu diesem Zweck. Nachteilig an diesem Verfahren ist die Tatsache, dass durch die Zugabe von Wasser eine unerwünschte Verseifungsreaktion auftreten kann, die die Biodieselausbeute verringern kann, wenn der alkalische Katalysator nicht zuvor neutralisiert wird.

CN 101423773 beschreibt die Zugabe von Ca- oder Mg-Salzen zu der Reaktionsmischung nach der Umesterung, ebenfalls mit dem Ziel, die Phasentrennung zu beschleunigen. Bei einigen der beschriebenen Salze kann es aufgrund der schlechten Löslichkeit zu Problemen mit unerwünschter Feststoffbildung kommen.

Sowohl bei der Wasserzugabe, als auch bei der Zugabe der Ca- oder Mg-Salze ist es nachteilig, dass das Additiv erst nach der Reaktion zugegeben wird, was zusätzlichen apparativen und zeitlichen Aufwand bedeutet.

Eine weitere Methode, die Biodieselherstellung unter Verwendung homogener Katalysatoren zu verbessern, ist die Verwendung von Co-Solventien, wie z. B. in Chemical Engineering Journal 2009, 146, 302; Energy&Fuels 2008, 22, 2702, oder Biomass&Bioenergy 1996, 11, 43 beschrieben.

Diese Co-Solventien beschleunigen zwar die Reaktion, dafür verschlechtern oder verhindern sie jedoch die Phasentrennung zur Abtrennung des Glycerins. Darüber hinaus müssen die Co-Solventien aufwendig aus dem Biodiesel und dem Glycerin entfernt werden.

Es war deshalb die Aufgabe der vorliegenden Erfindung ein vereinfachtes Verfahren zur Umesterung von Glyceriden mit einwertigen Alkoholen bereitzustellen, das eine schnellere und vollständigere Reaktion bewirkt.

Überraschenderweise wurde gefunden, dass Mehrkomponenten-Katalysatoren, d. h. Gemische aus verschiedenen Katalysatoren, bzw. herkömmliche Katalysatoren mit geeigneten Zusätzen, entweder die Umesterungsreaktion beschleunigen und/oder die Phasentrennung verbessern.

Demgemäß ist ein erster Gegenstand der vorliegenden Erfindung die Verwendung eines Katalysatorsystems umfassend einen Umesterungskatalysator, ausgewählt aus der Gruppe der Alkalimetall- oder Erdalkalimetall-alkanolate oder der Alkalimetallhydroxide, und mindestens einem vom Umesterungskatalysator verschiedenen Aktivator, ausgewählt aus der Gruppe umfassend salzartige Verbindungen, Titanate oder nicht salzartige Verbindungen mit einer Dichte von mindestens 0.9 g/mL, zur Katalyse von Umesterungsreaktionen.

Gegenüber den Katalysatoren gemäß dem Stand der Technik, die nur eine Komponente enthalten, haben die beschriebenen Mehrkomponenten-Katalysatoren den Vorteil, dass die Umesterungsreaktion und/oder die Phasentrennung des freigesetzten Glycerins beschleunigt wird und damit ein schnellerer und vollständigerer Prozess und/oder eine vereinfachte Biodieselaufarbeitung erzielt wird. Insbesondere die schnellere Phasentrennung stellt einen erheblichen Vorteil dar, weil somit die Biodiesel-Herstellung zusätzlich rationalisiert werden kann. Die eingesetzten Katalysatorsysteme bewirken eine schnellere und vollständigere Phasentrennung des freigesetzten Glycerins, weil die sich bildende Glycerinphase eine höhere Dichte und/oder eine größere Polarität hat.

Dabei wird das Katalysatorsystem als Ganzes bei der Umesterung eingesetzt, eine aufwendige spätere Zugabe von Additiven ist nicht notwendig.

Das erfindungsgemäße eingesetzte Katalysatorsystem weist mindestens zwei Komponenten auf, den Umesterungskatalysator und mindestens einen Aktivator.

Der Umesterungskatalysator ist für die eigentliche Umesterung verantwortlich und ist ausgewählt aus der Gruppe der Alkalimetall- oder Erdalkalimetall-alkanolate oder der Alkalimetallhydroxide. Bevorzugte Umesterungskatalysatoren sind Natrium-methanolat, Kalium-methanolat, Natrium-ethanolat, Kalium-ethanolat, Natrium-hydroxid oder Kalium-hydroxid. Ganz besonders bevorzugt werden Natrium-methanolat oder Kalium-methanolat als Umesterungskatalysatoren eingesetzt.

Üblicherweise liegen die Umesterungskatalysatoren in Lösung vor, insbesondere handelt es sich um alkoholische Lösungen, vorzugsweise um methanolische oder ethanolische Lösungen. Ganz besonders bevorzugt entspricht der eingesetzte Alkohol dem eingesetzten Alkanolat. Somit wird insbesondere Natrium-methanolat in Methanol oder Kalium-methanolat in Methanol als Umesterungskatalysator eingesetzt.

Zusätzlich enthält das Katalysatorsystem mindestens einen vom Umesterungskatalysator verschiedenen Aktivator. Der genannte Aktivator ist ausgewählt aus der Gruppe umfassend salzartige Verbindungen, Titanate oder nicht salzartige Verbindungen mit einer Dichte von mindestens 0.9 g/mL.

Als salzartige Verbindungen im Sinne der vorliegenden Erfindung werden Verbindungen verstanden, die ein Kation und ein Anion aufweisen. Insbesondere handelt es sich hierbei um Chloride, Bromide, Fluoride, Acetate, Formiate, Phosphate, Hydrogenphosphate, Sulfate, Hydrogensulfate, Nitrate, Carbonate, Hydrogencarbonate, Cyanide, Cyanate, Thiocyanate, Borate, Silikate, Aluminate, Alkanolate oder Hexacyanoferrate von Natrium, Kalium, Magnesium, Calcium, Zink oder Eisen. Der Begriff Alkanolate umfasst die entsprechenden Methanolate, Ethanolate, n-Propanolate, iso-Propanolate, tert-Butanolate oder tert-Pentanolate. Bevorzugt werden Methanolate und Ethanolate eingesetzt, ganz besonders bevorzugt Methanolate.

Ebenfalls eingesetzt werden können Titanate, insbesondere Tetramethyltitanat Ti(OMe)₄, Tetraethyltitanat Ti(OEt)₄, oder Tetraisopropyltitanat Ti(O-iso-Pr)₄.

Weiterhin eignen sich nicht salzartige Verbindungen mit einer Dichte von mindestens 0.9 g/mL. Die Dichte wird dabei nach dem Fachmann allgemein bekannten Methoden, z. B. mittels des Aräometerverfahrens (z. B. DIN EN ISO 3675) oder Pyknometerverfahrens, bestimmt.

Bei den genannten Verbindungen handelt es sich um organische Verbindungen, bevorzugt handelt es sich um Ethylenglycol, Diethylenglycol, Formamid, Dimethylformamid, N-Methylformamid, Acetamid, Dimethylacetamid, N-Methylacetamid, N-Ethylacetamid, Propanamid, N-Methylpropanamid, N-Ethylpropanamid, N-Methylpyrrolidon und/oder Dimethylsulfoxid, ganz besonders bevorzugt handelt es sich um Dimethylformamid.

Von den genannten Aktivatoren werden ganz besonders bevorzugt Kalium-methanolat, Kalium-formiat, Kaliumphosphat oder Dimethylformamid eingesetzt. Die genannten Aktivatoren haben den Vorteil, dass sie preisgünstig, gut verfügbar und in den eingesetzten Konzentrationen gut in der entstehenden Glycerinphase löslich sind.

Wesentlich für das eingesetzte Katalysatorsystem ist, dass der Umesterungskatalysator und der Aktivator voneinander verschieden sind. Dies betrifft insbesondere die erfindungsgemäße Ausführungsform bei der sowohl der Umesterungskatalysator als auch der Aktivator ein Alkalimetall- oder Erdalkalimetallalkanolat ist. Wird Natrium-methanolat als Umesterungskatalysator eingesetzt, so kann Kalium-methanolat als Aktivator eingesetzt werden. Umgekehrt ist der Einsatz von Natrium-methanolat als Aktivator denkbar, wenn Kalium-methanolat als Umesterungskatalysator eingesetzt wird.

Ganz besonders bevorzugte Katalysatorsysteme umfassen Natrium-methanolat mit Kalium-methanolat, Natrium-methanolat mit Kalium-formiat, Natrium-methanolat mit Dimethylformamid, Kalium-methanolat mit Kalium-formiat sowie Kalium-methanolat mit Dimethylformamid.

Die genannten Katalysatorsysteme eignen sich zum Einsatz bei Umesterungsreaktionen, wobei es grundsätzlich keine Einschränkungen hinsichtlich der Art der Umesterungsreaktion gibt. Als Umesterungsreaktion im Sinne der vorliegenden Erfindung wird eine Reaktion verstanden, bei der ein Eduktester und ein Alkohol in Gegenwart des Katalysatorsystems miteinander umgesetzt werden, so dass der Alkohol mit der Säurekomponente des Eduktesters zu einem entsprechend neuen Produktester reagiert, wobei die Alkoholkomponente des Eduktesters freigesetzt wird. Vorzugsweise wird das Katalysatorsystem zur Herstellung von Fettsäurealkylestern durch Umesterung von Mono-, Di- oder Triglyceriden eingesetzt. Dabei werden Mono-, Di- oder Triglyceride in die entsprechenden Fettsäurealkylester umgewandelt und gleichzeitig wird freies Glycerin erhalten.

Demgemäß ist ein weiterer Gegenstand der vorliegenden Erfindung ein Verfahren zur Herstellung von Fettsäurealkylestern, umfassend die Umesterung mindestens eines Mono-, Di- oder Triglycerids in Anwesenheit mindestens eines einwertigen Alkohols, dadurch gekennzeichnet, dass ein Katalysatorsystem umfassend einen Umesterungskatalysator, ausgewählt aus der Gruppe der Alkalimetall- oder Erdalkalimetall-alkanolate oder der Alkalimetallhydroxide, und mindestens einem vom Umesterungskatalysator verschiedenen Aktivator, ausgewählt aus der Gruppe umfassend salzartige Verbindungen, Titanate oder nicht salzartige Verbindungen mit einer Dichte von mindestens 0.9 g/mL zur Katalyse eingesetzt wird.

Die einsetzbaren Katalysatorsysteme sind bereits vorab genannt.

Ausgangsstoffe für das erfindungsgemäße Verfahren sind Mono-, Di- und Triglyceride der allgemeinen Formel (I) 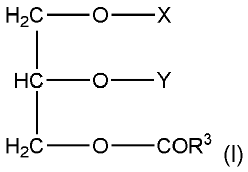 worin X = COR¹ oder H, Y = COR² oder H ist und R¹, R² und R³, die gleich oder verschieden sein können, aliphatische Kohlenwasserstoff-Gruppen mit 3 bis 23 C-Atomen bedeuten, wobei diese Gruppen gegebenenfalls mit einer OH-Gruppe substituiert sein können oder beliebige Gemische aus solchen Glyceriden.

Somit können in Glyceriden gemäß Formel (I) ein oder zwei Fettsäureester durch Wasserstoff ersetzt sein. Die Fettsäureester R¹CO-, R²CO- und R³CO- leiten sich ab von Fettsäuren mit 3 bis 23 Kohlenstoffatomen in der Alkylkette. R¹ und R² oder R¹, R² und R³ können in der obengenannten Formel gleich oder verschieden sein, wenn es sich um Di- oder Trigyceride handelt. Die Reste R¹, R² und R³ gehören den folgenden Gruppen an:
a) Alkylradikale, die verzweigt sein können, jedoch vorzugsweise geradkettig sind und 3 bis 23, vorzugsweise 7 bis 23 C-Atome haben;
b) olefinisch ungesättigte aliphatische Kohlenwasserstoffreste, die verzweigt sein können, jedoch vorzugsweise geradkettig sind, und die 3 bis 23, vorzugsweise 11 bis 21 und insbesondere 15 bis 21 C-Atome besitzen und die 1 bis 6, vorzugsweise 1 bis 3 Doppelbindungen enthalten, welche konjugiert oder isoliert sein können;
c) monohydroxy-substituierte Reste des Typs a) und b), vorzugsweise olefinisch ungesättigte Olefinreste, die 1 bis 3 Doppelbindungen besitzen, insbesondere den Rest der Ricinolsäure.

Die Acylradikale R¹CO-, R²CO- und R³CO- solcher Glyceride, die als Ausgangsmaterialien für das Verfahren der vorliegenden Erfindung geeignet sind, leiten sich ab von den folgenden Gruppen aliphatischer Carbonsäuren (Fettsäuren):
a) Alkansäuren oder deren alkylverzweigte, insbesondere methylverzweigte Derivate, die 4 bis 24 Kohlenstoffatome besitzen, wie zum Beispiel Buttersäure, Valeriansäure, Capronsäure, Heptansäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachidinsäure, Behensäure, Lignocerinsäure, 2-Methylbutansäure, Isobuttersäure, Isovaleriansäure, Pivalinsäure, Isocapronsäure, 2-Ethylcapronsäure, die stellungsisomeren Methylcaprinsäuren, Methyllaurinsäuren und Methylstearinsäuren, 12-Hexylstearinsäure, Isostearinsäure oder 3,3-Dimethylstearinsaure.
b) Alkensäuren, Alkadiensäuren, Alkatriensäuren, Alkatetraensäuren, Alkapentaensäuren und Alkahexaensäuren sowie deren alkylverzweigte, speziell methylverzweigte Derivate mit 4 bis 24 C-Atomen, wie zum Beispiel Crotonsäure, Isocrotonsäure, Caproleinsäure, 3-Lauroleinsäure, Myristoleinsäure, Palmitoleinsäure, Ölsäure, Elaidinsäure, Erucasäure, Brassidinsäure, 2,4-Decadiensäure, Linolsäure, 11,14-Eicosadiensäure, Eleostearinsäure, Linolensäure, Pseudoeleostearinsäure, Arachidonsäure, 4,8,12,15,18,21-Tetracosahexaensäure oder trans-2-Methyl-2-butensäure.
c₁) Monohydroxyalkansäuren mit 4 bis 24 C-Atomen, vorzugsweise mit 12 bis 24 C-Atomen, vorzugsweise unverzweigt, wie zum Beispiel Hydroxybuttersäure, Hydroxyvaleriansäure, Hydroxycapronsäure, 2-Hydroxydodecansäure, 2-Hydroxytetradecansäure, 15-Hydroxypentadecansäure, 16-Hydroxyhexadecansäure, Hydroxyoctadecansäure.
c₂) Ferner Monohydroxyalkensäuren mit 4 bis 24, vorzugsweise mit 12 bis 22 und insbesondere mit 16 bis 22 C-Atomen (vorzugsweise unverzweigt) und mit 1 bis 6, vorzugsweise mit 1 bis 3 und insbesondere mit einer ethylenischen Doppelbindung, wie zum Beispiel Ricinolsäure oder Ricinelaidinsäure.

Bevorzugte Ausgangsstoffe für das erfindungsgemäße Verfahren sind vor allem die natürlichen Fette, die Gemische aus überwiegend Triglyceriden und kleinen Anteilen aus Diglyceriden und/oder Monoglyceriden darstellen, wobei auch diese Glyceride meist wiederum Gemische darstellen und verschiedenartige Fettsäurereste im obengenannten Bereich, insbesondere solche mit 8 und mehr C-Atomen, enthalten. Beispielsweise seien genannt pflanzliche Fette, wie Olivenöl, Kokosfett, Palmkernfett, Babassuöl, Palmöl, Palmkernöl, Erdnußöl, Rapsöl (Rüböl), Ricinusöl, Sesamöl, Sonnenblumenöl, Sojaöl, Hanföl, Mohnöl, Avocadoöl, Baumwollsaatöl, Weizenkeimöl, Maiskeimöl, Kürbiskernöl, Tabaköl, Traubenkernöl, Jatrophaöl, Algenöl, Karanjaöl (Öl der Pongamia pinnata), Camelinaöl (Leindotteröl), Kakaobutter oder auch Pflanzentalge, ferner tierische Fette, wie Rindertalg, Schweinefett, Hühnerfett, Knochenfett, Hammeltalg, Japantalg, Walöl und andere Fischöle sowie Lebertran. Ebenso eingesetzt werden können aber auch einheitliche Tri-, Di- und Monoglyceride, sei es, dass diese aus natürlichen Fetten isoliert oder auf synthetischem Wege gewonnen wurden. Hier seien beispielsweise genannt: Tributyrin, Tricapronin, Tricaprylin, Tricaprinin, Trilaurin, Trimyristin, Tripalmitin, Tristearin, Triolein, Trielaidin, Trilinoliin, Trilinolenin, Monopalmitin, Monostearin, Monoolein, Monocaprinin, Monolaurin, Monomyristin oder gemischte Glyceride, wie beispielsweise Palmitodistearin, Distearoolein, Dipalmitoolein oder Myristopalmitostearin.

Unter einwertigen Alkoholen im Sinne der vorliegenden Erfindung werden Alkohole mit nur einer OH-Gruppe verstanden. Beispiele einwertiger Alkohole sind Methanol, Ethanol, n-Propanol, iso-Propanol und n-Butanol, iso-Butanol, sec-Butanol oder tert-Butanol, sowie verzweigte oder längerkettige, gegebenenfalls ebenfalls verzweigte Alkohole, wie beispielsweise Amylalkohol, tert-Amylalkohol, n-Hexanol und/oder 2-Ethylhexanol. Bevorzugt eingesetzt werden Methanol und Ethanol. Die genannten Alkohole können allein oder in Mischungen in dem erfindungsgemäßen Verfahren eingesetzt werden.

Die Konzentration des Umesterungskatalysators beträgt 0,001-20 Gew.-%, bevorzugt 0,01 - 5 Gew.-% und besonders bevorzugt 0,1-2 Gew.-%, bezogen auf die eingesetzte Menge an Mono-, Di- oder Triglycerid.

Die eingesetzte Menge an Aktivator beträgt 0,01 - 30 Gew.-%, bevorzugt 0,1 - 20 Gew.- % und ganz besonders bevorzugt 1-15 Gew.-%, bezogen auf die eingesetzte Menge an Umesterungskatalysator.

Das erfindungsgemäße Verfahren kann nach allen dem Fachmann bekannten Arten durchgeführt werden. Bei der Durchführung des erfindungsgemäßen Verfahrens wird die Reaktionsmischung vorzugsweise gerührt. Die bevorzugte intensive Durchmischung der Reaktionsmischung kann aber auch durch andere dem Fachmann geläufige Methoden erreicht werden.

Die Reaktionszeit wird vorzugsweise im Bereich von 1 bis 120 Minuten gewählt. Dabei werden Umsetzungsgrade von mindestens 98 %, vorzugsweise mindestens 99 % erreicht. Der Umsetzungsgrad der Reaktion bezieht sich auf den nach dem Ende oder dem Abbruch der Reaktion noch vorhanden Anteil an Glyceriden (= Summe von Tri-, Di- und Monoglyceriden) bezogen auf den Anfangsgehalt dieser Komponenten im eingesetzten Öl oder Fett. Der Umsatzgrad lässt sich in einfacher Weise gaschromatographisch bestimmen und errechnet sich aus den Gehalten der Alkylester geteilt durch die Summe der Gehalte von Alkylestern plus Glyceriden. Die nach dem erfindungsgemäßen Verfahren erhältlichen Fettsäurealkylester können als Biodiesel verwendet werden. Laut Spezifikation in DIN EN 14214 darf Biodiesel maximal 0,2 % Triglyceride nach Prüfverfahren EN 14105 enthalten. Bei herkömmlichen Umesterungsverfahren, die äquimolare Mengen NaOH, jedoch keine weiteren Aktivatoren einsetzen, werden Umsetzungsgrade der Größenordnung > 99,8 % erst nach längerer Zeit erzielt. Eine Erhöhung der NaOH-Konzentration zur Steigerung der Reaktionsgeschwindigkeit bei diesen herkömmlichen Umesterungsverfahren ist nicht wünschenswert, da NaOH dazu neigt Mono-, Di-oder Triglyceride, oder die entsprechenden Alkylester zu verseifen, und die korrespondierenden Seifen zu bilden, die einerseits Produktverluste bewirken und auch emulgierend wirken. Eine Phasentrennung nach beendeter Reaktion zur Trennung der Alkylesterphase und Glycerinphase wird dadurch erschwert bzw. verhindert. Eine Aufarbeitung des Produktes ist dann nur schwer möglich.

Das erfindungsgemäße Verfahren kann batchweise oder kontinuierlich (z. B. in einem Rohrreaktor, Rührkessel, Rührkesselkaskade, oder andere dem Fachmann bekannten Verfahren) durchgeführt werden.

Vorzugsweise stellt man bei dem erfindungsgemäßen Verfahren ein molares Verhältnis (einwertiger Alkohol : Mono-, Di-, Triglycerid) im Bereich von 3 : 1 bis 20 : 1 ein. Ein Verhältnis von 4:1 bis 8:1 ist dabei ganz besonders bevorzugt.

Das Katalysatorsystem wird vorzugsweise als Lösung in dem verwendeten einwertigen Alkohol eingesetzt, wobei der eigentliche Umesterungskatalysator vollständig gelöst ist, während der Aktivator in komplett oder auch nur teilweise gelöster Form vorliegen kann.

Die Umesterung wird in einem Temperaturbereich von 0 - 200°C, bevorzugt bei 10 - 100 °C und besonders bevorzugt bei 20 - 80 °C durchgeführt.

Dabei wird die Umesterung in einem Druckbereich von 0,1-100 bar, bevorzugt bei 0,5 - 50 bar und ganz besonders bevorzugt bei 1 - 5 bar durchgeführt.

Das Katalysatorsystem wird mit dem Mono-, Di- oder Triglycerid und gegebenenfalls zusätzlichem einwertigem Alkohol gemischt, wobei der einwertige Alkohol verbraucht und Glycerin freigesetzt wird. Wesentlich ist, dass das gesamte Katalysatorsystem, das heißt der Umesterungskatalysator und der Aktivator, als Mischung zu Beginn der Umesterungsreaktion vorliegt. Der eingesetzte Umsetzungskatalysator und der Aktivator verteilen sich größtenteils in der entstehenden schwereren Glycerinphase.

Die Reaktionsmischung kann auf unterschiedliche Weise aufgearbeitet werden. Nachdem man die Umesterung bis zum gewünschten Umsetzungsgrad, vorzugsweise bis 98 % oder höher durchgeführt hat, bilden sich in der Regel je eine Fettsäurealkylester- und eine Glycerin-Phase, die vom Fachmann durch bekannte Verfahrensschritte, wie beispielsweise Dekantieren, leicht getrennt werden können. Durch das erfindungsgemäße Katalysatorsystem wird die Trennung der Phasen beschleunigt, was die Aufarbeitung wesentlich erleichtert und die Raum-ZeitAusbeute erhöht.

Ein weiterer Erfindungsgegenstand ist die Verwendung der nach dem Verfahren erhältlichen Fettsäurealkylester als Bestandteil von Biodiesel (z. B. nach Spezifikation DIN EN 14214).

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen. Nachfolgend wird die vorliegende Erfindung anhand von Beispielen näher erläutert. Alternative Ausführungsformen der vorliegenden Erfindung sind in analoger Weise erhältlich.

### Beispiele:

Beispiel 1 bzw. 1b (nicht erfindungsgemäß):
500 g Algenöl (ca. 0,57 mol), 58 g Methanol (1,81 mol) und 7 g einer 30%igen methanolischen Lösung von Natrium-methanolat (0,04 mol Umesterungskatalysator) werden auf 60 °C erhitzt, gemischt und für eine Stunde gerührt.
Anschließend wird die Mischung in einen Scheidetrichter gegeben und die Zeit gemessen, bis zu der eine klare untere Glycerinphase aufgetreten ist. Im beschriebenen Experiment dauerte dies 8,15 min bzw. 8,54 min.
Beispiel 2-20 (erfindungsgemäß):
500 g Algenöl (ca. 0,57 mol), 58 g Methanol (1,81 mol) und 7 g einer 30%igen methanolischen Lösung von Natrium-methanolat (0,04 mol Umesterungskatalysator), welche verschiedene Aktivatoren enthält, werden auf 60 °C erhitzt, gemischt und für eine Stunde gerührt.
Anschließend wird die Mischung in einen Scheidetrichter gegeben und die Zeit gemessen, bis zu der eine klare untere Glycerinphase aufgetreten ist.
Die Ergebnisse können Tabelle 1 entnommen werden.

**Tabelle 1**

| Bsp. | Aktivator | Konzentration | Zeit f Phasentrennung |
|---|---|---|---|
| | | [%] | [min,sec] |
| 1 | - | | 8,45 |
| 1b | - | | 8,15 |
| 2 | Kaliumchlorid (KCl) | 15 | 4,30 |
| 3 | Kaliumchlorid (KCI) | 30 | 5,00 |
| 4 | Kalium-methanolat (KOMe) | 11 | 5,00 |
| 5 | Kalium-methanolat (KOMe) | 17 | 4,00 |
| 6 | Kalium-ethanolat (KOEt) | 12 | 6,00 |
| 7 | Kalium-t-butanolat (KOt-Bu) | 12 | 5,00 |
| 8 | Kalium-nitrat | 12 | 5,30 |
| 9 | Kaliumcarbonat (K₂CO₃) | 12 | 6,30 |
| 10 | Rubidiumchlorid (RbCl) | 12 | 6,00 |
| 11 | Kaliumacetat (KOAc) | 12 | 6,30 |
| 12 | Kaliumformiat (KO₂CH) | 12 | 4,30 |
| 13 | K₃[Fe(CN)₆] | 12 | 3,30 |
| 14 | K₄[Fe(CN)₆] | 12 | 5,30 |
| 15 | Cäsiumchlorid (CsCl) | 12 | 4,30 |
| 15 | Kaliumphosphat (K₃PO₄) | 12 | 4,30 |
| 16 | Kaliumthiocyanat (KSCN) | 12 | 5,00 |
| 17 | Ethylenglycol | 12 | 4,00 |
| 18 | Dimethylformamid | 12 | 3,30 |
| 19 | Propionamid | 12 | 4,00 |
| 20 | Glycerin | 30 | 4,10 |

Beispiel 21 (nicht erfindungsgemäß):
500 g Rapsöl (ca. 0,57 mol), 58 g Methanol (1,81 mol) und 8,5 g einer 32%igen methanolischen Lösung von Kalium-methanolat (0,04 mol Umesterungskatalysator) werden auf 60 °C erhitzt, gemischt und für eine Stunde gerührt.
Anschließend wird die Mischung in einen Scheidetrichter gegeben und die Zeit gemessen, bis zu der eine klare untere Glycerinphase aufgetreten ist. Im beschriebenen Experiment dauerte dies 8 min.
Beispiel 22 - 24 (erfindungsgemäß):
500 g Rapsöl (ca. 0,57 mol), 58 g Methanol (1,81 mol) und 8,5 g einer 32%igen methanolischen Lösung von Kalium-methanolat (0,04 mol Umesterungskatalysator), welche verschiedene Aktivatoren enthält, werden auf 60 °C erhitzt, gemischt und für eine Stunde gerührt.
Anschließend wird die Mischung in einen Scheidetrichter gegeben und die Zeit gemessen, bis zu der eine klare untere Glycerinphase aufgetreten ist.
Die Ergebnisse können Tabelle 2 entnommen werden.

**Tabelle 2**

| Bsp. | Aktivator | Konzentration | Zeit f Phasentrennung |
|---|---|---|---|
| | | [%] | [min,sec] |
| 21 | - | | 8,00 |
| 22 | Rubidiumchlorid (RbCl) | 11 | 3,30 |
| 23 | Kaliumchlorid (KCI) | 11 | 4,00 |
| 24 | Ethylenglycol | 11 | 4,00 |

Beispiel 25 (nicht erfindungsgemäß):
300 g Rapsöl (ca. 0,34 mol), 35 g Methanol (1,09 mol), welches 0,94 g (0,02 mol) NaOH enthält, werden auf 60 °C erhitzt, gemischt und für eine Stunde gerührt. Anschließend wird die Mischung in einen Scheidetrichter gegeben und die Zeit gemessen, bis zu der eine klare untere Glycerinphase aufgetreten ist. Im beschriebenen Experiment dauerte dies 18 Minuten.
Beispiel 26 (erfindungsgemäß):
300 g Rapsöl (ca. 0,34 mol), 35 g Methanol (1,09 mol), welches 0,94 g (0,02 mol) NaOH und 0,6 g einer 32%igen methanolischen Kalium-methanolatlösung enthält, werden auf 60 °C erhitzt, gemischt und für eine Stunde gerührt.
Anschließend wird die Mischung in einen Scheidetrichter gegeben und die Zeit gemessen, bis zu der eine klare untere Glycerinphase aufgetreten ist. Im beschriebenen Experiment dauerte dies 16 Minuten.
Beispiel 27 (nicht erfindungsgemäß):
300 g Rapsöl (ca. 0,34 mol), 35 g Methanol (1,09 mol), welches 1,50 g (0,02 mol) KOH enthält, werden auf 60 °C erhitzt, gemischt und für eine Stunde gerührt. Anschließend wird die Mischung in einen Scheidetrichter gegeben und die Zeit gemessen, bis zu der eine klare untere Glycerinphase aufgetreten ist. Im beschriebenen Experiment dauerte dies 21 Minuten.
Beispiel 28 (erfindungsgemäß):
300 g Rapsöl (ca. 0,34 mol), 35 g Methanol (1,09 mol) welches 1,50 g (0,02 mol) KOH und 0,6 g Rubidiumchlorid als Aktivator enthält, werden auf 60 °C erhitzt, gemischt und für eine Stunde gerührt.
Anschließend wird die Mischung in einen Scheidetrichter gegeben und die Zeit gemessen, bis zu der eine klare untere Glycerinphase aufgetreten ist. Im beschriebenen Experiment dauerte dies 19 Minuten.

In allen Beispielen ist eine deutliche Verkürzung der Zeit bis zur Phasenseparation zu beobachten, wenn ein gemäß der vorliegenden Erfindung einzusetzendes Katalysatorsystem verwendet wird.

## Patentansprüche

1. Verwendung eines Katalysatorsystems umfassend einen Umesterungskatalysator, ausgewählt aus der Gruppe der Alkalimetall- oder Erdalkalimetall-alkanolate oder der Alkalimetallhydroxide, und mindestens einem vom Umesterungskatalysator verschiedenen Aktivator, ausgewählt aus der Gruppe umfassend salzartige Verbindungen, Titanate oder nicht salzartige Verbindungen mit einer Dichte von mindestens 0.9 g/mL, zur Katalyse von Umesterungsreaktionen.

2. Verwendung gemäß Anspruch 1, **dadurch gekennzeichnet, dass** der Umesterungskatalysator Natrium-methanolat, Kalium-methanolat, Natrium-ethanolat, Kalium-ethanolat, Natrium-hydroxid oder Kalium-hydroxid ist.

3. Verwendung gemäß Anspruch 1 oder 2, **dadurch gekennzeichnet, dass** als salzartige Verbindungen Chloride, Bromide, Fluoride, Acetate, Formiate, Phosphate, Hydrogenphosphate, Sulfate, Hydrogensulfate, Nitrate, Carbonate, Hydrogencarbonate, Cyanide, Cyanate, Thiocyanate, Borate, Silikate, Aluminate, Alkanolate oder Hexacyanoferrate von Natrium, Kalium, Magnesium, Calcium, Zink oder Eisen eingesetzt werden.

4. Verwendung gemäß einem oder mehreren der Ansprüche 1 bis 3, **dadurch gekennzeichnet, dass** als Titanate Tetramethyltitanat, Tetraethyltitanat oder Tetraisopropyltitanat eingesetzt werden.

5. Verwendung gemäß einem oder mehreren der Ansprüche 1 bis 4, **dadurch gekennzeichnet, dass** als nicht salzartige Verbindungen mit einer Dichte von mindestens 0.9 g/mL Ethylenglycol, Diethylenglycol, Formamid, Dimethylformamid, N-Methylformamid, Acetamid, Dimethylacetamid, N-Methylacetamid, N-Ethylacetamid, Propanamid, N-Methylpropanamid, N-Ethylpropanamid, N-Methylpyrrolidon und/oder Dimethylsulfoxid eingesetzt werden.

6. Verwendung gemäß einem oder mehreren der Ansprüche 1 bis 5, **dadurch gekennzeichnet, dass** das Katalysatorsystem zur Herstellung von Fettsäurealkylestern durch Umesterung von Mono-, Di- oder Triglyceriden eingesetzt wird.

7. Verfahren zur Herstellung von Fettsäurealkylestern, umfassend die Umesterung mindestens eines Mono-, Di- oder Triglycerids in Anwesenheit mindestens eines einwertigen Alkohols, **dadurch gekennzeichnet, dass** ein Katalysatorsystem umfassend einen Umesterungskatalysator, ausgewählt aus der Gruppe der Alkalimetall- oder Erdalkalimetall-alkanolate oder der Alkalimetallhydroxide, und mindestens einem vom Umesterungskatalysator verschiedenen Aktivator, ausgewählt aus der Gruppe umfassend salzartige Verbindungen, Titanate oder nicht salzartige Verbindungen mit einer Dichte von mindestens 0.9 g/mL zur Katalyse eingesetzt wird.

8. Vefahren gemäß Anspruch 7, **dadurch gekennzeichnet, dass** der Umesterungskatalysator Natrium-methanolat, Kalium-methanolat, Natrium-ethanolat, Kalium-ethanolat, Natrium-hydroxid oder Kalium-hydroxid ist.

9. Verfahren gemäß Anspruch 7 oder 8, **dadurch gekennzeichnet, dass** die Konzentration des Umesterungskatalysators 0,001-20 Gew.-% beträgt, bezogen auf die eingesetzte Mono-, Di- oder Triglycerid-Menge.

10. Verfahren gemäß einem oder mehreren der Ansprüche 7 bis 9, **dadurch gekennzeichnet, dass** die Konzentration des Aktivators 0,01-25 Gew.-% beträgt, bezogen auf die Menge an Umesterungskatalysator.

11. Verfahren gemäß einem oder mehreren der Ansprüche 7 bis 10, **dadurch gekennzeichnet, dass** die Umesterung in einem Temperaturbereich von 0 bis 200 °C, durchgeführt wird.

12. Verfahren gemäß einem oder mehreren der Ansprüche 7 bis 11, **dadurch gekennzeichnet, dass** die Umesterung in einem Druckbereich von 0,1 -100 bar durchgeführt wird.

13. Verfahren gemäß einem oder mehreren der Ansprüche 7 bis 12, **dadurch gekennzeichnet, dass** als einwertiger Alkohol Methanol, Ethanol, n-Propanol, iso-Propanol, n-Butanol, iso-Butanol, sec-Butanol oder tert-Butanol, Amylalkohol, tert-Amylalkohol, n-Hexanol und/oder 2-Ethylhexanol eingesetzt wird.

14. Verwendung der nach dem Verfahren gemäß einem oder mehreren der Ansprüche 7 bis 13 hergestellten Fettsäurealkylester als Bestandteil von Biodiesel.
